# EUROPEAN PATENT APPLICATION

(11) **EP 0 970 696 A1**
(43) Date of publication of application: **12.01.2000**
(21) Application number: 98108127.6
(22) Date of filing: 05.05.1998
(51) Int. Cl.: A61K 31/52, A61K 31/34

(54) **Combination of loop diuretics with adenosine A1-receptor antagonists**

(71) Applicant: KYOWA HAKKO KOGYO CO., Ltd., Chiyoda-ku, Tokyo 100 (JP)
(72) Inventor: Hropot, Max, Dr., D-65349 Flörsheim am Main (DE)
(74) Representative: VOSSIUS & PARTNER

(57) **Abstract**

The invention relates to a combination of a loop diuretic and a adenosine A1 receptor antagonist; a pharmaceutical composition containing a loop diuretic and an adenosine A1-receptor antagonist; the preparation of a pharmaceutical composition which comprises bringing a mixture of a loop diuretic and an adenosine A1 receptor antagonist into a galenic administration form; the use of a combination of a loop diuretic and an adenosine A1-receptor antagonist or of a pharmaceutical composition containing a loop diuretic and an adenosine A1-receptor antagonist for the simultaneous, separate or sequential administration in the treatment and/or prophylaxis of hypertension, renal failure or disorders with increased proximal tubular reabsorption, e.g. congestive heart failure, liver cirrhosis or nephrotic syndrome.

## Description

Loop diuretics which specifically interacts with the Na⁺ K⁺ 2Cl⁻ co-transporter in the ascending limb of Henle's loop are very well established as a standard agent for the treatment of oedematous states associated with cardiac, renal and hepatic failure, and in treatment of hypertension with impaired renal function.

At the site of action loop diuretics such as furosemide inhibits the reabsorption of about 25% of filtered water and sodium, thus increasing distal tubular load delivery and reducing the transepithelial electrochemical gradient in the renal medulla. Because of the compensatory mechanism about 5% to 10% of filtered water and sodium are reabsorbed in distal tubules and collecting ducts, reducing the final fluid and sodium excretion after maximal effect of furosemide to about 15% to 20% of filtered load.

However, in severe cases of congestive heart failure, liver cirrhosis and in nephrotic syndrome an avid proximal tubular sodium and fluid reabsorption occurs resulting in significantly reduced distal fluid and NaCl load delivery. In these patients up to 90% of filtered load may be reabsorbed in the proximal tubule. Consequently, at the site of action of loop diuretics the amount of NaCl and fluid is dramatically diminished so that loop diuretics such as furosemide can not produce a considerable salidiuresis.

Salidiuresis can be increased by combined administration of a loop diuretic and a diuretic having a more distal tubular site of action (J. Furrer et al., 1980, Schweiz. Med. Wschr. 110, Nr. 48, 1825- 1829; Hropot et al., Kidney International, 1985, Vol. 28, 477-489). The well accepted standard treatment is the combination of furosemide with either metolazone or hydrochlorothiazide.

We now have found, surprisingly, that loop diuretics combined with adenosine A1-receptor antagonists potentiated saliduresis in rats with respect to NaCl and fluid excretion compared to single treatment while potassium excretion remained nearly constant resulting in favorable sodium/potassium ratios. Potassium loss is often detrimental to patients with congestive heart failure leading to life threatening arrhythmias.

Objects of the invention are therefore:
A combination of a loop diuretic and a adenosine A1 receptor antagonist;
a pharmaceutical composition containing a loop diuretic and an adenosine A1-receptor antagonist;
the preparation of a pharmaceutical composition which comprises bringing a mixture of a loop diuretic and an adenosine A1 receptor antagonist into a galenic administration form;
the use of a combination of a loop diuretic and an adenosine A1-receptor antagonist or of a pharmaceutical composition containing a loop diuretic and an adenosine A1-receptor antagonist for the simultaneous, separate or sequential administration in the treatment and/or prophylaxis of hypertension, renal failure or disorders with increased proximal tubular reabsorption, e.g. congestive heart failure, liver cirrhosis or nephrotic syndrome.

Adenosine A₁-receptor antagonists exert salidiuresis by antagonizing A1-receptor mediated sodium and bicarbonate reabsorption in the kidney. Adenosine receptors are cell surface G-protein-coupled receptors. Adenosine A1-receptors are coupled to a pertussis toxin-sensitive G-protein and inhibit cAMP stimulation induced by ligands such as catecholamines and histamine. Adenosine A1-receptor stimulation does not reduce the activity of adenylyl cyclase in the absence of stimulatory agonists. In the kidney, adenosine A1-receptors are expressed in the renal vasculature, the juxtaglomerular cells, proximal tubules, the thick ascending limb and the papillary collecting duct. Adenosine is produced in the kidney constitutively and regulates electrolyte reabsorption and renal haemodynamics. Reabsorption of sodium and bicarbonate from proximal tubules as well as chloride reabsorption in early distal tubules are under tonic adenosine stimulation mediated by A1-receptors. Furthermore, adenosine is an endogenous physiological antagonist of the renin-angiotensin system. In addition, adenosine A1-receptors mediate tubulo-glomerular feedback (TGF), a system by which the kidney decreases glomerular filtration in response to an increased NaCl delivery to the macula densa in early distal tubules.

Suitable adenosine A1-receptor antagonists for the purpose of the invention are: Xanthine derivatives such as xanthine amine congener (XAC); 8-Cyclopentyl-1,3-dipropylxanthine (DPCPX); 1,3-dipropyl-8-cyclopentyl-xanthine(CPX); 1,3-dipropyl-8-(3-noradamantyl)xanthine (NAX); 1,3-dipropyl-8[2(5,6-epoxy)norbonyl]xanthine (ENX); CVT-124; 1,3-dipropyl-8-(2-amino-4-chlorophenyl)-xanthine(PACPX); 3-[(4-amino)phenylethyl]-8-cyclopentylxanthine (BW-A844U); BW-A 522; 8-sulphophenyl-theophylline (SPT);8-Phenyltheophylline; 8-(noradamantan-3-yl)-1,3-dipropylxanthine (KW-3902); 1,3-dipropyl-8(dicyclopropylmethyl)xanthine (KF15372); (+,-)-8-(3-oxocyclopentyl)-1,3-dipropylxanthine (KFM 19); MDL 102,503; or non-Xanthine derivatives such as triazoloquinalozines as described in Francis et al., J. Med. Chem. 31, 1014-1020 (1988); N⁶-Endonorboran-2-yl-9-methyladenine (N-0861); N-Cyclopentyl-9-methyladenine (N-0840); N⁶-Butyl-8-phenyladenine; (R)-1-[(E)-3-(2-phenylpyrazolo[1,5-a]pyridin-3-yl]acryloyl]-2-piperidin-2-yl acetic acid (FK-352); 3-[2-(3-carboxypropyl)-3-oxo-2,3-dihydropiridazin-6-yl]-2-phenylpyrazolo[1,5-a]pyridine (FK-838); FK-453; FK 113453; WRC-0571 or physiologically tolerable salts thereof, as well as analouges of those compounds as are described in U.S. Patents Nos. 5,234,930; 4,985,444; 5,565,566; 5,066,655; 5,446,046; 5,395,836 or 5,290782.
Preferred are the compounds specifically named above. Especially preferred adenosine A1-receptor antagonists are KFM 19, N-0861, FK-352, FK-838, CVT-124 and KW-3902 or physiologically tolerable salts thereof. Most preferred adenosine A1-receptor antagonist is KW-3902 or a physiologically tolerable salt thereof.

Suitable loop diuretics for the purpose of the invention are:
Furosemide, bumetanide, piretanide, torasemide, azosemide, ethcrynic acid or etozoline which is a pro-drug of ozolinone or physiologically tolerable salts thereof. Preferred loop diuretics are furosemide and piretanide or physiologically tolerable salts thereof. Most preferred loop diuretic is furosemide or a physiologically tolerable salt thereof.

Preferred combinations according to the invention are those of KFM 19, N-0861, FK-352, FK-838, CVT-124 and KW-3902 or physiologically tolerable salts thereof with furosemide, bumetanide, piretanide and torasemide or physiologically tolerable salts thereof.

The most preferred combination according to the invention is that of KW-3902 or a physiologically tolerable salt thereof with furosemide or a physiologically tolerable salt thereof.

Physiologically tolerable salts of compounds according to the invention are understood as being both their organic and inorganic salts, such as are described in Remington's Pharmaceutical Sciences [17th Edition, page 1418 (1985)]. On account of the physical and chemical stability and the solubility, for acidic groups, inter alia, sodium, potassium, calcium and ammonium salts are preferred; for basic groups, inter alia, salts of hydrochloric acid, sulfuric acid, phosphoric acid or of carboxylic acids or sulfonic acids, such as, for example, acetic acid, citric acid, benzoic acid, maleic acid, fumaric acid, tartaric acid and p-toluene sulfonic acid, are preferred.

The combination, in accordance with the invention, can be used as an agent for the treatment and/or prophylaxis of hypertension, renal failure or disorders with increased proximal tubular reabsorption and therefore, is especially suited for the treatment of congestive heart failure (CHF), liver cirrhosis or nephrotic syndrome. Common to CHF, liver cirrhosis and nephrotic syndrome is the development of increased total body sodium and water content in the interstitial space. In general, edema occurs when there is an alteration of the Starling forces that control transfer of fluid from the vascular compartment to surrounding tissue space. The kidney plays an essential role in the retention of this sodium and water. Furthermore, water is often retained in excess of sodium in severe CHF and may lead to hemodilution and hyponatremia. Low cardiac output and a decreased effective arterial blood volume (EABV) in CHF are associated with a decrease in glomerular filtration rate (GFR) and compensatory increase in proximal tubular reabsorption which diminishes fluid delivery to the diluting segment (loop of Henle) in the distal nephron. The decreased EABV triggers the nonosmolar (baroreceptor) pathway for vasopressin release and increased sodium and fluid reabsorption in the proximal tubule, thereby diminishing distal delivery of fluid to the diluting segment.

In decompensated cirrhotic patients (with ascites and/or oedema) the EABV is diminished as a result of: a) portal hypertension, b) decreased total peripheral resistance, and c) decreased plasma oncotic pressure. The decrease in EABV stimulates the renal retention of salt and water by a fall in GFR and increased proximal tubular reabsorption in order to restore EABV to normal, thereby impair water excretion. Moreover, elevated levels of vasopressin relative to their plasma osmolality have been demonstrated in decompensated hyponatremic cirrhotic patients. The increase in renal sympathetic tone may activate the renin-angiotensin-aldosterone axis and thereby enhance the distal sodium reabsorption.

Oedema which develops in nephrotic syndrome is a consequence of an alteration in the Starling forces across capillary walls that results from proteinuria and hypoalbuminemia. As a consequence the EABV may be low in patients with nephrotic syndrome and stimulates the non osmotic release of vasopressin. The mechanism of renal sodium and water retention is quite similar to that which occurs in congestive heart failure and liver cirrhosis.

The combination, in accordance with the invention, can be administered orally or intravenously, preferably intravenously. Alternatively, such combination might be orally administered in a prophylactic manner to CHF patients NYHA III to IV to prevent or delay decompensation and/or to reduce the duration of hospitalization. Further indications are renal failure, liver cirrhosis, nephrotic syndrome and hypertension resistant to standard treatment.

Besides administration as a fixed combination, the invention also relates to the simultaneous, separate or sequential administration of a loop diuretic with an adenosine A1 receptor antagonist for the treatment and/or prophylaxis of the abovementioned diseases.

The invention furthermore relates to a commercial pack, comprising as pharmaceutical active compound a) a loop diuretic and b) an adenosine A1 receptor antagonist together with instructions for the use of these active compounds in combination for simultaneous, separate or sequential administration in the treatment or prophylaxis of the abovementioned diseases.

Preferred commercial packs are those which contain furosemide or a physiologically tolerable salt thereof as loop diuretic and KW-3902 or physiologically tolerable salt thereof as adenosine A1 receptor antagonist.

By combined administration, the effect of one combination component can be potentiated by the respective other component, i.e. the action and/or duration of action of a combination or preparation according to the invention is stronger or longer-lasting than the action and/or the duration of action of the respective individual components (additive and/or synergistic effects). In the case of combined administration, this leads to a lowering of the dose of the respective combination component, compared with individual administration. The combinations and preparations according to the invention accordingly have the advantage that the amounts of active compound to be administered can be significantly reduced and undesirable side effects can be eliminated or greatly reduced.

The pharmacologically utilizable combinations of the present invention and their salts can be used for the preparation of pharmaceutical products which contain an effective amount of the active substances together with vehicles, and which are suitable for enteral and parenteral administration. Tablets or gelatine capsules which contain the active compounds together with diluents, for example lactose, dextrose, sucrose, mannitol, sorbitol, cellulose and/or glycine, and lubricants such as diatomateous earth, talc, stearic acid or its salts, such as magnesium or calcium stearate, and/or polyethylene glycol. Tablets likewise contain binders, such as magnesium aluminium silicate, starch, gelatin, traganth, methyl cellulose, sodium carboxymethyl cellulose and/or polyvinylpyrrolidone, and, if necessary, pigment, flavorings and sweeteners.

Injectable solutions are preferably isotonic aqueous solutions, suspensions or lipid emulsions, most preferably lipid emulsions which may contain auxiliaries such as preservatives, stabilizing agents, wetting and/or emulsifying agents, solubilizers, salts to regulate the osmotic pressure and/or buffer substances.

The pharmaceutical compositions according to the invention can be prepared, for example, by either intensively mixing the individual components as powders, or by dissolving the individual components in the suitable solvents such as, for example, a lower alcohol and then removing the solvent.

The weight ratio of adenosine A1 receptor antagonist to the loop diuretic in the combinations and preparations according to the invention is expediently 1:0.01 to 1:100, preferably 1:0.1 to 1:10.

The combination and preparations according to the invention contain a total of preferably 0.5-99.5% by weight, in particular 4-99% by weight, of these active compounds.

When used according to the invention in mammals, preferably in man, the doses of the various active compound components vary, for example, in the range from 0.001 to 100 mg/kg/day, preferably from 0.01 to 30 mg/kg/day.

The examples which follow serve to illustrate the present invention without the latter being restricted to them.

### Example 1

### Salidiuretic effect of KW-3902 and furosemide in rats

Salidiuretic effect of KW-3902 and furosemide and their combinations were evaluated in male Wistar rats. Rats were fasted for 16 hours previous to the experiment but had free access to tap water. The animals were distributed at random in 4 groups of 3 rats each per dose and placed in diuresis cages. In addition to compounds rats received orally NaCl solution in a volume of 25 ml/kg body weight. The compound KW-3902 (suspended in gum arabic 0.5%) was administered orally in doses of 0.001, 0.01 and 0.1 mg/kg and furosemide (dissolved in NaCl solution) at oral doses of 10, 20 and 30 mg/kg each in a volume of 5 ml/kg body weight and compared with a control group. In addition, KW-3902 at the dose of 0.1 mg/kg was administered simultaneously with each furosemide dose used.

The excretion of urine and electrolytes was measured in the collection periods 1-4 hours after administration of the compound and vehicle. The urine volumes of each group and each observation period were collected separately. Sodium, potassium, and chloride were determined to assess the saluretic activity of the compound. Sodium and potassium were measured by flame photometry (Flame photometer, Eppendorf, Hamburg), chloride argentometrically by potentiometrical end point determination (Chloridmeter Eppendorf, Hamburg) and osmolality by freezing point reduction (Vogel osmometer, Giessen). The analytical results were used for calculation of excretion of urine (ml/kg b.wt.) and electrolytes (mmol/kg b.wt.). Results are indicated as arithmetical means and standard error of the mean. Statistics were performed with Mann-Whitney U-test.

Results of salidiuretic experiments are shown in table 1. KW-3902 in doses of 0.01 and 0.1 and furosemide in doses of 10, 20 and 30 mg/kg body weight orally caused a significant increase in urine volume, sodium and chloride excretion as compared to the control. The combinations of KW-3902 in a dose of 0.1 mg/kg with furosemide in doses of 10 mg/kg exerted significant additive or even synergistic effects with respect to urine volume, sodium and chloride excretion in the collection periods 1-4 hours as compared with single compounds. Potassium excretion remained nearly constant resulting in favorable sodium/potassium ratios. However, when furosemide was administered in doses of 20 and 30 mg/kg body weight simultaneously with KW-3902 at a dose of 0.1 mg/kg, additive or synergistic effects could not be elaborated due to high-ceiling effects of furosemide in normal rats.

### Example 2

### Salidiuretic effect of KW-3902 and furosemide in rats with liver cirrhosis

Salidiuretic effects of KW-3902 and furosemide and their combinations were evaluated in male Wistar rats with liver cirrhosis.
Liver cirrhosis was induced by oral treatment with carbon tetrachloride (CCl₄) as described previously (Bickel et al., Z. Gastroenterol. 34, 49-55, 1996). Briefly, animals received CCl₄, 1 ml/kg p.o., dissolved in olive oil 1:1 (v/v) twice weekly for 6 weeks. Rats were fasted for 16 hours previous to the diuretic experiment but had free access to tap water. The animals were distributed at random in groups of 7 rats per dose and placed in diuresis cages. In addition to compounds rats received orally NaCl solution in a volume of 25 ml/kg body weight. The compound KW-3902 was administered intravenously as fat emulsion in doses of 0.1 and 1.0 mg/kg and furosemide in a dose of 30 mg/kg orally and intravenously. The excretion of urine and electrolytes was measured in the collection periods 1-5 and 1-24 hours after administration of compounds and vehicle. The urine volumes of each group and each observation period were collected separately. Sodium, potassium, and chloride were determined as described in example 1. The analytical results were used for calculation of excretion of urine (ml/kg b.wt.) and electrolytes (mmol/kg b.wt.). Results are indicated as arithmetical means and standard error of the mean. Statistics were performed with Mann-Whitney U-test. Results of salidiuretic experiments are shown in table 2.
The compounds KW-3902 and furosemide exerted significant salidiuretic effects as compared to the control group. The combinations of KW-3902 in intravenous dose of 0.1 mg/kg with oral furosemide in a dose of 30 mg/kg exerted significant additive or synergistic effects with respect to urine volume, sodium and chloride excretion in the collection periods 1-5 and 1-24 hours as compared to single compounds. Potassium excretion remained nearly constant resulting in favorable sodium/potassium ratios. However, intravenous administration of furosemide at the dose of 30 mg/kg caused a high-ceiling effect in this animal model which was not exceeded by addition of KW-3902 at the dose of either 0,1 or 1 mg/kg intravenously.

### Example 3

### Preparation of an oral combination product containing KW-3902 and furosemide

KW-3902 (30 g) is dissolved in a mixed solvent of ethanol (350 g) and dichloromethane (350 g), and Eudragit L 100 (methacylic acid-methacrylic acid methyl ester copolymer; Röhm GmbH) (90 g) is added to the solution. The resulting solution is sprayed on the mixture of furosemide (120 g) and lactose (760 g), and granulated at 60°C with a fluidized bed granulator. After drying, the granules are sieved (24 mesh) and mixed uniformly and homogeneously with magnesium stearate in a blender. The resulting granules are filled into hard gelatin capsules (size 2). The capsules containing 5 mg of KW-3902 and 20mg of furosemide are obtained.

### Example 4

### Preparation of a parenteral combination product of KW-3902 and furosemide

Purified egg yolk lecithin (25 g), soy bean oil (25 g), oleic acid (1.2 g), conc. glycerol (11.05 g) and distilled water (387.5 ml) are mixed by a Vacuum Emulsifier (PVQS-1, Mizuho Industry Co., Ltd.) To achieve a rough emulsification. KW-3902 (250 mg) and furosemide (500 mg) are added to this mixture, and dissolved in the emulsion by vigorously mixing at 60°C for 30 min. Prefiltration of this preparation is conducted using Omnipore Membrane (pore size: 10 µm, diameter: 142 mm, Millipore Co., Ltd.). The emulsification of this preparation is performed by passing 10 times at an emulsifying pressure of 1,000 kg/cm² in a Microfluidizer (M-110-EH, Mizuho Industry Co., Ltd.). The pH value of the lipid emulsion is adjusted at 7.0 with 1/50N sodium hydroxide, and the distilled water is added to the lipid emulsion preparation up to the total volume of 500 ml. After sterile filtration through a membrane filter (DFA3201NRP, Pall Co., Ltd.), 2 ml each of the lipid emulsion is dispensed to ampoules. The atmosphere of the ampoules is substituted with nitrogen gas, and ampoules are closed by tightly winding the aluminum cap.

### List of abbreviations:

- b.wt.: body weight
- Cl⁻: chloride ion
- g: gram
- g/mol: gram per mol
- K⁺: potassium ion
- mmol/kg: millimole per kilogram
- mg/kg: milligram per kilogram
- ml/kg: milliliter per kilogram
- mosmol/kg: milliosmol per kilogram
- Na⁺: sodium ion
- SEM: standard error of the mean

**Table 1**

| Salidiuretic effects of KW-3902, Furosemide and their combinations orally in rats, 1 - 4 hours collection period (n=4 groups of 3 rats, mean ± SEM) | | | | | | |
|---|---|---|---|---|---|---|
| | Urine ml/kg | Na+ | K+ mmol/kg | Cl- | Osmolality mosmol/kg | Na⁺/K⁺ |
| NaCl 25 ml/kg | 25.49 | 1.92 | 1.46 | 2.56 | 12.69 | 1.31 |
| | 0.36 | 0.11 | 0.04 | 0.07 | 0.55 | |
| KW-3902 0.001 mg/kg | 18.09 | 1.75 | 0.93 | 2.19 | 10.00 | 1.88 |
| | 1.74 | 0.23 | 0.20 | 0.25 | 1.46 | |
| KW-3902 0.01 mg/kg | 26.72 | 2.42 | 1.32 | 2.82 | 13.60 | 1.84 |
| | 2.38 | 0.14 | 0.18 | 0.09 | 0.23 | |
| KW-3902 0.1 mg/kg | 45.56 | 4.91 | 1.74 | 5.24 | 18.88 | 2.81 |
| | 3.37 | 0.20 | 0.13 | 0.22 | 1.11 | |
| Furosemide 10 mg/kg | 32.27 | 3.11 | 1.34 | 4.30 | 13.34 | 2.32 |
| | 0.84 | 0.20 | 0.02 | 0.16 | 0.60 | |
| Furosemide 20 mg/kg | 60.72 | 6.51 | 1.73 | 8.30 | 21.03 | 3.76 |
| | 2.30 | 0.46 | 0.07 | 0.41 | 1.27 | |
| Furosemide 30 mg/kg | 71.70 | 8.20 | 1.71 | 10.13 | 23.63 | 4.73 |
| | 3.97 | 0.41 | 0.07 | 0.49 | 1.06 | |
| Furosemide 10 + KW 0.1 | 58.80 | 6.51 | 1.63 | 7.70 | 20.50 | 3.99 |
| | 1.91 | 0.22 | 0.08 | 0.22 | 0.33 | |
| Furosemide 20 + KW 0.1 | 70.68 | 7.82 | 1.72 | 9.39 | 22.51 | 4.53 |
| | 1.95 | 0.32 | 0.07 | 0.37 | 0.46 | |
| Furosemide 30 + KW 0.1 | 78.39 | 9.08 | 2.02 | 10.71 | 26.07 | 4.50 |
| | 5.50 | 0.69 | 0.23 | 0.70 | 2.61 | |

**Table 2**

| Salidiuretic effects of KW-3902, furosemide and their combinations in rats with liver cirrhosis (n=7 rats, mean ± SD) | | | | | | | |
|---|---|---|---|---|---|---|---|
| | | Urine ml/kg | Na⁺ | K⁺ mmol/kg | Cl⁻ | Osmolality mosmol/kg | Na⁺/K⁺ |
| Control | 1 - 5 hrs | 16.92 | 1.09 | 0.78 | 1.69 | 9.97 | 1.41 |
| | | 5.35 | 0.28 | 0.39 | 0.41 | 2.27 | |
| | 1 - 24 hrs | 39.61 | 4.27 | 2.60 | 4.23 | 33.92 | 1.65 |
| | | 12.79 | 1.37 | 0.66 | 1.24 | 5.86 | |
| KW-3902 0.1 mg/kg i.v. | 1 - 5 hrs | 31.18 | 3.67 | 1.08 | 4.36 | 15.31 | 3.39 |
| | | 5.99 | 0.85 | 0.19 | 0.48 | 1.35 | |
| | 1 - 24 hrs | 39.67 | 5.03 | 2.14 | 5.20 | 29.17 | 2.36 |
| | | 6.33 | 0.92 | 0.29 | 0.48 | 3.14 | |
| KW-3902 1 mg/kg i.v. | 1 - 5 hrs | 37.94 | 4.57 | 1.35 | 5.10 | 17.48 | 3.39 |
| | | 3.60 | 0.50 | 0.23 | 0.63 | 1.81 | |
| | 1 - 24 hrs | 46.88 | 6.16 | 2.54 | 6.21 | 32.19 | 2.43 |
| | | 3.49 | 0.41 | 0.41 | 0.52 | 3.18 | |
| Furosemide 30 mg/kg p.o. | 1 - 5 hrs | 29.03 | 3.14 | 0.93 | 4.55 | 12.38 | 3.39 |
| | | 7.37 | 1.04 | 0.52 | 1.56 | 5.50 | |
| | 1 - 24 hrs | 43.88 | 4.77 | 2.62 | 5.27 | 33.79 | 1.82 |
| | | 15.56 | 1.81 | 1.21 | 1.56 | 11.52 | |
| Furosemide 30 mg/kg i.v. | 1 - 5 hrs | 64.55 | 7.57 | 1.61 | 10.01 | 20.85 | 4.71 |
| | | 15.94 | 1.92 | 0.23 | 2.45 | 4.92 | |
| | 1 - 24 hrs | 76.67 | 8.72 | 4.05 | 10.30 | 40.58 | 2.15 |
| | | 15.97 | 1.80 | 0.51 | 2.46 | 6.42 | |
| KW 0.1 i.v. + FU 30 p.o. | 1 - 5 hrs | 52.32 | 6.29 | 1.14 | 8.12 | 20.19 | 5.54 |
| | | 16.39 | 2.12 | 0.29 | 2.10 | 5.84 | |
| | 1 - 24 hrs | 62.58 | 7.30 | 2.50 | 8.48 | 36.59 | 2.92 |
| | | 16.58 | 2.12 | 0.66 | 2.08 | 3.67 | |
| KW 0.1 i.v. + FU 30 i.v. | 1 - 5 hrs | 70.14 | 8.34 | 1.79 | 10.03 | 22.67 | 4.65 |
| | | 6.63 | 1.08 | 0.20 | 1.11 | 2.10 | |
| | 1 - 24 hrs | 78.66 | 9.30 | 3.98 | 10.31 | 37.79 | 2.34 |
| | | 6.11 | 0.93 | 0.81 | 1.08 | 5.68 | |
| KW 1 i.v. + FU 30 i.v. | 1 - 5 hrs | 73.24 | 8.83 | 1.69 | 11.12 | 24.08 | 5.23 |
| | | 14.70 | 2.01 | 0.20 | 2.09 | 0.18 | |
| | 1 - 24 hrs | 79.51 | 9.42 | 4.16 | 10.82 | 40.74 | 2.26 |
| | | 13.39 | 1.61 | 0.29 | 1.25 | 5.13 | |

## Claims

1. A pharmaceutical composition comprising
a) a loop diuretic, and
b) an adenosine A1 receptor antagonist.

2. A pharmaceutical composition according to claim 1, wherein said adenosine A1 receptor antagonist is selected from the group consisting of xanthine amine congener (XAC); 8-Cyclopentyl-1,3-dipropylxanthine (DPCPX); 1,3-dipropyl-8-cyclopentyl- xanthine (CPX); 1,3-dipropyl-8-(3-noradamantyl)xanthine (NAX); 1,3-dipropyl-8[2(5,6-epoxy)norbonyl]xanthine (ENX); CVT-124; 1,3-dipropyl-8-(2-amino-4-chlorophenyl)-xanthine (PACPX); 3-[(4-amino)phenylethyl]-8-cyclopentylxanthine (BW-A844U); BW-A 522; 8-sulpho-phenyl-theophylline (SPT);8-Phenyltheophylline; 8-(noradamantan-3-yl)-1,3-dipropylxanthine (KW-3902); 1,3-dipropyl-8(dicyclopropylmethyl)xanthine (KF15372); (+,-)-8-(3-oxocyclopentyl)-1,3-dipropylxanthine (KFM 19); MDL 102,503;) N⁶-Endonorboran-2-yl-9-methyladenine (N-0861); N-Cyclopentyl-9-methyladenine (N-0840); N⁶-Butyl-8-phenyladenine; N⁶-Butyl-8-phenyladenine; (R)-1-[(E)-3-(2-phenylpyrazolo[1,5-a]pyridin-3-yl]acryloyl]-2-piperidin-2-yl acetic acid (FK-352); 3-[2-(3-carboxypropyl)-3-oxo-2,3-dihydropiridazin-6-yl]-2-phenylpyrazolo[1,5-a]pyridine (FK-838); FK-453; FK 113453 or WRC-0571; or a physiologically tolerable salt thereof.

3. A pharmaceutical composition according to claim 1, wherein said adenosine A1 receptor antagonist is KFM 19, N-0861, FK-352, FK-838, CVT-124 or KW-3902; or a physiologically tolerable salt thereof.

4. A pharmaceutical composition according to claim 1, wherein said adenosine A1 receptor antagonist is KW-3902; or a physiologically tolerable salt thereof.

5. A pharmaceutical composition according to claims 1 to 4, wherein said loop diuretic is selected from the group consisting of furosemide, bumetanide, piretanide, torasemide, azosemide, ethcrynic acid or etozoline; or a physiologically tolerable salt thereof.

6. A pharmaceutical composition according to claims 1 to 4, wherein said loop diuretic is selected from the group consisting of furosemide, bumetanide, piretanide or torasemide; or a physiologically tolerable salt thereof.

7. A pharmaceutical composition according to claims 1 to 4, wherein said loop diuretic is furosemide, or a physiologically tolerable salt thereof.

8. A pharmaceutical composition according to claim 1 containing KW-3902 or a physiologically tolerable salt thereof and furosemide or a physiologically tolerable salt thereof.

9. A pharmaceutical composition according to claims 1 to 8 for the treatment and/or prophylaxis of hypertension.

10. A pharmaceutical composition according to claims 1 to 8 for the treatment and/or prophylaxis of renal failure.

11. A pharmaceutical composition according to claims 1 to 8 for the treatment and/or prophylaxis of disorders with increased proximal tubular reabsorption.

12. A pharmaceutical composition according to claim 11, wherein said disorder is congestive heart failure, liver cirrhosis or nephrotic syndrome.

13. The use of a loop diuretic in combination with an adenosine A1 receptor antagonist for the production of a pharmaceutical composition for the treatment and/or prophylaxis of hypertension or renal failure.

14. The use of a loop diuretic in combination with an adenosine A1 receptor antagonist for the production of a pharmaceutical for the treatment and/or prophylaxis of disorders with increased proximal tubular reabsorption.

15. The use according to claim 14, wherein said disorder is congestive heart failure, liver cirrhosis or nephrotic syndrome.

16. The use according to claims 13 to 15, wherein said adenosine A1 receptor antagonist is selected from the group consisting of xanthine amine congener (XAC); 8-Cyclopentyl-1,3-dipropylxanthine (DPCPX); 1,3-dipropyl-8-cyclopentyl- xanthine (CPX); 1,3-dipropyl-8-(3-noradamantyl)xanthine (NAX); 1,3-dipropyl-8[2(5,6-epoxy)norbonyl]xanthine (ENX); CVT-124; 1,3-dipropyl-8-(2-amino-4-chlorophenyl)-xanthine (PACPX); 3-[(4-amino)phenylethyl]-8-cyclopentylxanthine (BW-A844U); BW-A 522;8-sulphophenyl-theophylline (SPT);8-Phenyltheophylline; 8-(noradamantan-3-yl)-1,3-dipropylxanthine (KW-3902); 1,3-dipropyl-8(dicyclopropylmethyl)xanthine (KF15372); (+,-)-8-(3-oxocyclopentyl)-1,3-dipropylxanthine (KFM 19);, MDL 102,503;) N⁶-Endonorboran-2-yl-9-methyladenine (N-0861); N-Cyclopentyl-9-methyladenine (N-0840); N⁶-Butyl-8-phenyladenine; N⁶-Butyl-8-phenyladenine; (R)-1-[(E)-3-(2-phenylpyrazolo[1,5-a]pyridin-3-yl]acryloyl]-2-piperidin-2-yl acetic acid (FK-352); 3-[2-(3-carboxypropyl)-3-oxo-2,3-dihydropiridazin-6-yl]-2-phenylpyrazolo[1,5-a]pyridine (FK-838); FK-453; FK 113453 or WRC-0571; or a physiologically tolerable salt thereof.

17. The use according to claims 13 to 15, wherein said adenosine A1 receptor antagonist is KFM 19, N-0861, FK-352, FK-838, CVT-124 or KW-3902; or a physiologically tolerable salt thereof.

18. The use according to claims 13 to 15, wherein said adenosine A1 receptor antagonist is KW-3902; or a physiologically tolerable salt thereof.

19. The use according to claims 13 to 18, wherein said loop diuretic is selected from the group consisting of furosemide, bumetanide, piretanide, torasemide, azosemide, ethcrynic acid or etozoline; or a physiologically tolerable salt thereof.

20. The use according to claims 13 to 18, wherein said loop diuretic is selected from the group consisting of furosemide, bumetanide, piretanide or torasemide; or a physiologically tolerable salt thereof.

21. The use according to claims 13 to 18, wherein said loop diuretic is furosemide, or a physiologically tolerable salt thereof.

22. The use according to claims 13 to 15, wherein said adenosine A1 receptor antagonist is KW-3902 or a physiologically tolerable salt thereof and said loop diuretic is furosemide or a physiologically tolerable salt thereof.
